# EUROPÄISCHE PATENTSCHRIFT

(11) **EP 0 691 157 B1**
(45) Veröffentlichungstag und Bekanntmachung des Hinweises auf die Patenterteilung: **25.08.1999**
(21) Anmeldenummer: 95109869.8
(22) Anmeldetag: 24.06.1995
(51) Int. Cl.: B01J 23/72, B01J 23/76, C07C 209/60

(54) **Verfahren zur Herstellung von Aminen**
Process for preparing amines
Procédé pour la préparation d'amines

(30) Priorität: 04.07.1994 DE 4423346
(43) Veröffentlichungstag der Anmeldung: 10.01.1996
(73) Patentinhaber: BASF Aktiengesellschaft, 67063 Ludwigshafen (DE)
(72) Erfinder: Reif, Wolfgang, Dr., D-67227 Frankenthal (DE); Hesse, Michael, Dr., D-67105 Schifferstadt (DE); Zimmermann, Horst, Dr., D-68199 Mannheim (DE); Lingk, Heinz, D-67240 Bobenheim-Roxheim (DE)

(56) Entgegenhaltungen:
- EP-A- 0 382 049
- EP-A- 0 484 800
- DE-A- 4 021 230
- GB-A- 2 164 034

## Beschreibung

Die vorliegende Erfindung betrifft den Einsatz von Katalysatoren, die im wesentlichen aus Kupferoxid und Zirkonoxid und gegebenenfalls Metalloxide der Nebengruppen Ib bis VIIb und VIII des Periodensystems der Elemente bestehen, zur Herstellung von Aminen aus Nitrilen und Stickstoffverbindungen bei erhöhten Temperaturen und Drücken.

Aus Archiv der Pharmazie 271, (1933), 439 bis 445 ist bekannt, daß bei der Reduktion von Nitrilen neben primären Aminen häufig auch die entsprechenden sekundären und tertiären Amine entstehen. Zur Herstellung gemischter sekundärer Amine kann bei der Hydrierung von Nitrilen auch ein primäres oder sekundäres Amin zugesetzt werden. Die beschriebenen Umsetzungen wurden jedoch mit teuren Palladiumkatalysatoren durchgeführt und dabei nur schlechte Raum-Zeit-Ausbeuten erzielt.

Aus JP 50/47910 und JP 50/47911 ist bekannt, daß sekundäre bzw. tertiäre Ethylamine durch Umsetzung von Acetonitril mit Ethylamin an Raney-Kobalt oder Raney-Nickel hergestellt werden können. Die beschriebene diskontinuierliche Verfahrensweise ermöglicht jedoch ebenfalls nur unbefriedigende Raum-Zeit-Ausbeuten.

Der vorliegenden Erfindung lag daher Aufgabe zugrunde, den zuvor genannten Nachteilen abzuhelfen.

Demgemäß wurde ein Verfahren zur Herstellung von Aminen aus Nitrilen und Stickstoffverbindungen, ausgewählt aus der Gruppe Ammoniak, primäre und sekundäre Amine, bei Temperaturen von 80 bis 250°C und Drücken von 1 bis 400 bar mit Wasserstoff in Gegenwart eines Katalysators bestehend aus 85 bis 100 Gew.-% Kupferoxid und Zirkonoxid und 15 bis 0 Gew.-% Metalloxide der Nebengruppen Ib bis VIIb und VIII des Periodensystems der Elemente gefunden.

Die im erfindungsgemäßen Verfahren eingesetzten Kupferoxid/Zirkonoxid-Katalysatoren bestehen aus 85 bis 100 Gew.-% Kupferoxid und Zirkonoxid und 15 bis 0 Gew.-% Metalloxide der Nebengruppen Ib bis VIIb und VIII des Periodensystems der Elemente, bevorzugt aus 90 bis 100 Gew.-% Kupferoxid und Zirkonoxid und 10 bis 0 Gew.-% Metalloxide der Nebengruppen Ib bis VIIb und VIII des Periodensystems der Elemente, besonders bevorzugt aus 95 bis 100 Gew.-% Kupferoxid und Zirkonoxid und 5 bis 0 Gew.-% Metalloxide der Nebengruppen Ib bis VIIb und VIII des Periodensystems der Elemente, insbesonders aus 98 bis 100 Gew.-% Kupferoxid und Zirkonoxid und 2 bis 0 Gew.-% Metalloxide der Nebengruppen Ib bis VIIb und VIII des Periodensystems der Elemente, ganz besonders aus 99,5 bis 100 Gew.-% Kupferoxid und Zirkonoxid und 0,5 bis 0 Gew.-% Metalloxide der Nebengruppen Ib bis VIIb und VIII des Periodensystems der Elemente.

Das erfindungsgemäße Verfahren kann wie folgt durchgeführt werden:

Die Umsetzung erfolgt im allgemeinen ohne zusätzliches Lösungsmittel. Bei der Umsetzung hochmolekularer hochviskoser oder bei Raumtemperatur fester Ausgangsverbindungen oder Produkte kann es vorteilhaft sein, ein unter den Reaktionsbedingungen inertes Lösungsmittel, wie Tetrahydrofuran, Dioxan, Wasser oder Ethylenglykoldimethylether mitzuverwenden.

Üblicherweise arbeitet man bei der Umsetzung bei Temperaturen von 80 bis 230°C, bevorzugt 90 bis 170°C, besonders bevorzugt 100 bis 140°C. Im allgemeinen wird die Reaktion bei einem Druck von 1 bis 400 bar ausgeführt. Bevorzugt werden jedoch Drücke von 10 bis 250 bar, insbesondere von 30 bis 200 bar angewandt.

Die Anwendung höherer Temperaturen und eines höheren Gesamtdruckes ist möglich. Der Gesamtdruck im Reaktionsgefäß, welches sich aus der Summe der Partialdrücke des Aminierungsmittels, des Nitrils und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck eingestellt.

Der Wasserstoff wird der Reaktion im allgemeinen in einer Menge von 5 bis 400Nl, bevorzugt in einer Menge von 50 bis 200Nl pro Mol Nitrilkomponente zugeführt.

Es kann für die Selektivität des vorliegenden Verfahrens vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, daß man dem Katalysator, der sich üblicherweise in einem bevorzugt von außen beheizten Festbettreaktor befindet, bei der gewünschten Reaktionstemperatur und dem gewünschten Druck das Nitril und das Aminierungsmittel simultan zuführt. Dabei belastet man den Katalysator im allgemeinen mit 0,02 bis 2, bevorzugt 0,05 bis 1 und besonders bevorzugt mit 0,1 bis 0,8 l Nitril pro Liter Katalysator und Stunde.

Der Reaktor kann sowohl in der Sumpf- als auch in der Rieselfahrweise betrieben werden, d.h. man kann die Reaktanten sowohl von unten nach oben als auch von oben nach unten durch den Reaktor leiten. Es versteht sich von selbst, daß sich das Verfahren sowohl diskontinuierlich als auch kontinuierlich durchführen läßt. In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Als Aminierungsmittel bei der hydrierenden Aminierung von Nitrilen können Ammoniak, bevorzugt primäre oder sekundäre, aliphatische oder cycloaliphatische Amine eingesetzt werden.

Bevorzugt werden diese Aminierungsmittel zur Herstellung unsymmetrisch substituierter Di- oder Trialkylamine, wie Ethyldiisopropylamin und Ethyldicyclohexylamin verwendet. Bevorzugt werden beispielsweise die folgenden Mono- und Dialkylamine als Aminierungsmittel verwendet: Methylamin, Dimethylamin, Ethylamin, Diethylamin, Propylamin, Diisopropylamin, Butylamin, Pentylamin, Hexylamin und Cyclohexylamin.

Das Aminierungsmittel kann bezüglich der zu aminierenden Nitrilgruppe in stöchiometrischer Menge eingesetzt werden. Bevorzugt wird jedoch mit einem Überschuß an Aminierungsmittel gearbeitet und zwar im allgemeinen mit einem mehr als 2 molaren Überschuß pro Mol zu aminierender Nitrilgruppe. Es wird im allgemeinen mit einem 2 bis 50-fachen, bevorzugt 5 bis 25-fachen, insbesondere 10 bis 20-fachen molaren Überschuß pro Mol umzusetzender Nitrilgruppen eingesetzt. Höhere Überschüsse an primären oder sekundären Aminen sind möglich.

Aus dem Reaktionsaustrag können nachdem dieser zweckmäßigerweise entspannt worden ist, das überschüssige Aminierungsmittel und der Wasserstoff entfernt und die erhaltenen aminierten Produkte durch Destillation, Flüssigextraktion oder Kristallisation gereinigt werden. Das überschüssige Aminierungsmittel und der Wasserstoff werden vorteilhaft wieder in die Reaktionszone zurückgeführt. Das gleiche gilt für die eventuell nicht oder nicht vollständig umgesetzte Nitrilkomponente.

Im allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, daß man die katalytisch aktive, zu Pulver vermahlene Masse in das Reaktionsgefäß einbringt oder, daß man die katalytisch aktive Masse nach Mahlung, Vermischung mit Formhilfsmitteln, Formung und Temperung als Katalysatorformkörper - beispielsweise als Kugeln, Zylinder, Ringe, Spiralen - im Reaktor anordnet.

Da sich die Konzentrationsangaben jeweils - falls nicht anders angegeben - auf die katalytisch aktive Masse des Katalysators beziehen, wird die katalytisch aktive Masse des Katalysators im folgenden als die Summe der Massen der katalytisch aktiven Bestandteile im Katalysator, jeweils berechnet als Oxide nach dessen letzter Wärmebehandlung und vor dessen Reduktion mit Wasserstoff, definiert.

Zur Herstellung der Vollkatalysatoren sind verschiedene Verfahrensweisen möglich. Sie sind beispielsweise durch Anteigen pulvriger Mischungen der Hydroxide, Carbonate, Oxide und/oder anderer Salze der Komponenten Zirkonium und Kupfer und gegebenenfalls eines oder mehrerer Metalloxide der Nebengruppen Ib bis VIIb oder VIII des Periodensystems der Elemente mit Wasser und nachfolgendes Extrudieren und Tempern der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Elemente enthaltenden, wäßrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden, vorteilhaft werden diese Aufschlämmungen durch Ausfällen der schwerlöslichen Zirkoniumverbindungen aus wäßrigen Zirkoniumsalzlösungen mittels Mineralbasen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wäßrige Salzlösung in der Wärme und unter Rühren so lange mit einer wäßrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderlich, gute Wasserlöslichkeit. Es wird als selbstverständlich erachtet, daß bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäße Katalysatoren mit besonders vorteilhaften Eigenschaften sind dadurch erhältlich, daß man einen Teil der Zirkoniumkomponente des Katalysators, zweckmäßigerweise aus einer wäßrigen Zirkoniumsalzlösung, separat in einer Fällungsapparatur durch Zugabe wäßriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde.

Für das erfindungsgemäße Verfahren erweist es sich in der Regel als besonders zweckmäßig, Katalysatoren bestehend aus 95 bis 100 Gew.-% Kupferoxid und Zirkonoxid und 5 bis 0 Gew.-% Metalloxide der Nebengruppen Ib bis VIIb und VIII des Periodensystems der Elemente, bevorzugt aus 98 bis 100 Gew.-% Kupferoxid und Zirkonoxid und 2 bis 0 Gew.-% Metalloxide der Nebengruppen Ib bis VIIb und VIII des Periodensystems der Elemente, besonders bevorzugt aus 99,5 bis 100 Gew.-% Kupferoxid und Zirkonoxid und 0,5 bis 0 Gew.-% Metalloxide der Nebengruppen Ib bis VIIb und VIII des Periodensystems der Elemente, insbesonders aus 100 Gew.-% Kupferoxid und Zirkonoxid, einzusetzen.

Das Innenverhältnis von Kupferoxid zu Zirkonoxid enthält üblicherweise 30 bis 70 Gew.-% sauerstoffhaltige Kupferverbindungen, bevorzugt 40 bis 60 Gew.-% sauerstoffhaltige Kupferverbindungen, besonders bevorzugt 45 bis 55 Gew.-% sauerstoffhaltige Kupferverbindungen, ganz besonders bevorzugt 49 bis 53 Gew.-% sauerstoffhaltige Kupferverbindungen.

Die bei diesen Fällungsreaktionen enthaltenen Niederschläge sind im allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200°C, vorzugsweise bei 100 bis 150°C, getrocknet.

Nach der Trocknung wird der Katalysator zweckmäßigerweise konditioniert, sei es, daß man ihn durch Vermahlen auf eine bestimmte Korngröße einstellt oder daß man ihn nach seiner Vermahlung mit Formhilfsmitteln wie Graphit oder Stearinsäure vermischt, mittels einer Tablettenpresse zu Formlingen verpreßt und tempert. Die Tempertemperaturen entsprechen dabei im allgemeinen den Temperaturen bei der Trocknung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und gegebenenfalls gehandelt. Vor ihrem Einsatz als Katalysatoren zur Herstellung von Aminen aus Nitrilen werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so daß diese gemeinsam mit den verschiedenartigen Sauerstoffverbindungen in der aktiven Form das Katalysators vorliegen.

Die Substituenten R¹, R², R³, R⁴, R⁵ und die Indizes l, m und n in den Verbindungen I, II und III haben unabhängig voneinander folgende Bedeutungen:
R¹, R², R⁴, R⁵
   - Wasserstoff, bevorzugt R¹ Wasserstoff, wenn R² ungleich Wasserstoff ist,
R³
   - C₁- bis C₂₀₀-Alkyl, bevorzugt C₁- bis C₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀- bis C₂₀₀-Alkyl wie Polybutyl, Polyisobutyl, Polypropyl, Polyisopropyl und Polyethyl, besonders bevorzugt Polybutyl und Polyisobutyl,
R¹, R², R³
   - C₃- bis C₁₂-Cycloalkyl, bevorzugt C₃- bis C₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇- bis C₂₀-Alkylaryl, bevorzugt C₇- bis C₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇- bis C₂₀-Aralkyl, bevorzugt C₇- bis C₁₂-Phenylalkyl, wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R³
   - C₁- bis C₂₀-Hydroxyalkyl, bevorzugt C₁- bis C₈-Hydroxyalkyl, besonders bevorzugt C₁- bis C₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methyl-ethyl,
   - durch Amino und/oder Methylamino und/oder Dimethylamino und Hydroxy substituiertes C₁- bis C₂₀-Alkyl, bevorzugt durch Amino und/oder Methylamino und/oder Dimethylamino und/oder Hydroxy substituiertes C₁- bis C₈-Alkyl, besonders bevorzugt durch Amino und/oder Methylamino und/oder Dimethylamino und/oder Hydroxy substituiertes C₁- bis C₄-Alkyl, wie N-(Hydroxyethyl)aminoethyl und N-(Aminoethyl)aminoethyl,
   - C₂- bis C₃₀-Alkoxyalkyl, bevorzugt C₂- bis C₂₀-Alkoxyalkyl, besonders bevorzugt C₂- bis C₈-Alkoxyalkyl, wie Methoxymethyl, Ethoxymethyl, n- Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxy-ethyl, besonders bevorzugt C₂- bis C₄-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
   - R⁴-(OCHR⁵CH₂)ₙ,
R¹ und R²
   - C₁- bis C₂₀-Alkyl, bevorzugt C₁- bis C₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
R⁴
   - C₁- bis C₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
R⁵
   - Methyl,
X
   - Sauerstoff,
   - N-R⁵,
l
   - eine ganze Zahl von 2 bis 4 wie 2, 3 und 4, bevorzugt 2 und 3, besonders bevorzugt 2,
m
   - eine ganze Zahl von 1 bis 4 wie 1, 2, 3 und 4, bevorzugt 2, 3 und 4, besonders bevorzugt 2 und 3,
n
   - eine ganze Zahl von 2 bis 10, bevorzugt eine ganze Zahl von 2 bis 8 wie 2, 3, 4, 5, 6, 7 oder 8, besonders bevorzugt eine ganze Zahl von 2 bis 6 wie 2, 3, 4, 5 oder 6.

Die erfindungsgemäß erhältlichen Amine eignen sich u.a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

### Beispiele

### Katalysatorherstellung

Lösung 1: Zirkonacetat und Kupfernitrat werden in destilliertem Wasser gelöst, so daß 27,4 Liter einer Lösung entstehen, die 2,4 kg Zirkon (berechnet als ZrO₂) und 5,25 kg Kupfer (berechnet als CuO) enthält. Der pH-Wert der Lösung wird durch HNO₃ auf ca. 1 gestellt.

Lösung 2: Natriumcarbonat wird in destilliertem Wasser so gelöst, daß eine 20 Gew.-% Soda enthaltende Lösung entsteht.

In 60 Liter Wasser werden 2,4 kg Zirkondioxid aufgeschlämmt und die Lösung auf 80°C aufgewärmt. In diese gut gerührte Aufschlämmung werden die Lösungen 1 und 2 parallel so zugegeben, daß der pH-Wert im Fallbehälter bei 5,5 bleibt. Nach vollendeter Zugabe der Lösung 1 wird der pH-Wert durch Zugabe von Lösung 2 auf 7,5 hochgestellt.

Die so erhaltene Fällmaische wird auf einer Filterpresse gewaschen und entwässert. Das resultierende Pulver wird bei 120°C 20 h getrocknet.

Das so erhaltene Pulver wird mit 3 % Graphit vermischt und zu Tabletten mit einem Durchmesser von 5 mm verpreßt.

Die resultierenden Tabletten haben eine chemische Zusammensetzung von 52,6 Gew.-% CuO und 47,4 Gew.-% ZrO₂.

### Beispiel 1 Methylalkylierung von N-Methylaminopropionitril

Ein kontinuierlich betriebener Hochdruckreaktor wurde mit 500 cm³ Katalysator gefüllt und stündlich mit 40 cm³ N-Methylaminopropionitril und 400 cm³ flüssigem Monomethylamin beschickt. Die Katalysatortemperatur wurde auf 130°C und der Druck im Reaktor, durch gleichzeitiges Aufpressen von Wasserstoff, auf 200 bar eingestellt. Aus dem Reaktionsaustrag wurde nach dessen Entspannung überschüssiges Monomethylamin abdestilliert. Die GC-Analyse ergab vollständigen Umsatz und folgende Produktzusammensetzung:
8,3 % N-Methylpropylendiamin
74,3 % N,N'-Dimethylpropylendiamin
17,3 % sonstige Nebenprodukte

### Beispiel 2

Ein kontinuierlich betriebener Hochdruckreaktor wurde mit 500 cm³ Katalysator gefüllt und stündlich mit 100 cm³ N-Methylaminopropionitril und 400 cm³ flüssigem Monomethylamin beschickt. Die Katalysatortemperatur wurde auf 130°C und der Druck im Reaktor, durch gleichzeitiges Aufpressen von Wasserstoff, auf 200 bar eingestellt. Aus dem Reaktionsaustrag wurde nach dessen Entspannung überschüssiges Monomethylamin abdestilliert. Die GC-Analyse ergab >98 %igen Umsatz und folgende Produktzusammensetzung:
3,9 % N-Methylpropylendiamin
68,6 % N,N'-Dimethylpropylendiamin
25,2 % sonstige Nebenprodukte

## Patentansprüche

1. Verfahren zur Herstellung von Aminen aus Nitrilen und Stickstoffverbindungen, ausgewählt aus der Gruppe Ammoniak, primäre und sekundäre Amine, bei Temperaturen von 80 bis 250°C und Drücken von 1 bis 400 bar mit Wasserstoff in Gegenwart eines Katalysators, dadurch gekennzeichnet, daß man einen Katalysator bestehend aus 85 bis 100 Gew.-% Kupferoxid und Zirkonoxid und 15 bis 0 Gew.-% Metalloxide der Nebengruppen Ib bis VIIb und VIII des Periodensystems der Elemente einsetzt.

2. Verfahren nach Anspruch 1, dadurch gekennzeichnet, daß der Katalysator aus 90 bis 100 Gew.-% Kupferoxid und Zirkonoxid und 10 bis 0 Gew.-% Metalloxide der Nebengruppen Ib bis VIIb und VIII des Periodensystems der Elemente besteht.

3. Verfahren nach Anspruch 1, dadurch gekennzeichnet, daß der Katalysator aus 95 bis 100 Gew.-% Kupferoxid und Zirkonoxid und 5 bis 0 Gew.-% Metalloxide der Nebengruppen Ib bis VIIb und VIII des Periodensystems der Elemente besteht.

4. Verfahren nach Anspruch 1, dadurch gekennzeichnet, daß der Katalysator aus 98 bis 100 Gew.-% Kupferoxid und Zirkonoxid und 2 bis 0 Gew.-% Metalloxide der Nebengruppen Ib bis VIIb und VIII des Periodensystems der Elemente besteht.

5. Verfahren nach Anspruch 1, dadurch gekennzeichent, daß der Katalysator aus 99,5 bis 100 Gew.-% Kupferoxid und Zirkonoxid und 0,5 bis 0 Gew.-% Metalloxide der Nebengruppen Ib bis VIIb und VIII des Periodensystems der Elemente besteht.

6. Verfahren nach den Ansprüchen 1 bis 5 zur Herstellung von Aminen der allgemeinen Formel I 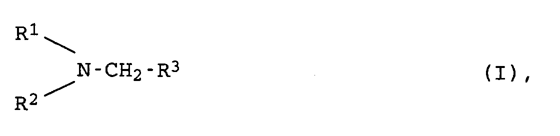 in der
R¹ und R² Wasserstoff, C₁- bis C₂₀-Alkyl, C₃- bis C₁₂-Cycloalkyl, Aryl, C₇- bis C₂₀-Aralkyl und C₇- bis C₂₀-Alkylaryl,
R³ C₁- bis C₂₀₀-Alkyl, C₃- bis C₁₂-Cycloalkyl, C₁- bis C₂₀-Hydroxyalkyl, durch Amino und/oder Methylamino und/oder Dimethylamino und/oder Hydroxy substituiertes C₁- bis C₂₀-Alkyl, C₂- bis C₃₀-Alkoxyalkyl, R⁴-(OCHR⁵CH₂)ₙ, Aryl, C₇- bis C₂₀-Aralkyl und C₇- bis C₂₀-Alkylaryl,,
R⁴ Wasserstoff, C₁- bis C₄-Alkyl,
R⁵ Wasserstoff oder Methyl,
x Sauerstoff oder N-R⁵,
n eine ganze Zahl von 2 bis 30,
l eine ganze Zahl von 2 bis 4,
m eine ganze Zahl von 1 bis 4
bedeuten, aus Nitrilen der allgemeinen Formel II
R³-CN (II)
und Stickstoffverbindungen der allgemeinen Formel III

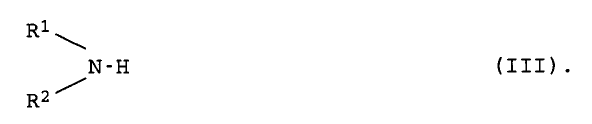

## Claims

1. A process for preparing amines from nitriles and nitrogen compounds selected from the group consisting of ammonium, primary and secondary amines at temperatures of from 80 to 250°C and pressures of from 1 to 400 bar using hydrogen in the presence of a catalyst, wherein the catalyst used comprises from 85 to 100% by weight of copper oxide and zirconium oxide and from 15 to 0% by weight of metal oxides of transition groups Ib to VIIb and VIII of the Periodic Table of the Elements.

2. A process as claimed in claim 1, wherein the catalyst comprises from 90 to 100% by weight of copper oxide and zirconium oxide and from 10 to 0% by weight of metal oxides of transition groups Ib to VIIb and VIII of the Periodic Table of the Elements.

3. A process as claimed in claim 1, wherein the catalyst comprises from 95 to 100% by weight of copper oxide and zirconium oxide and from 5 to 0% by weight of metal oxides of transition groups Ib to VIIb and VIII of the Periodic Table of the Elements.

4. A process as claimed in claim 1, wherein the catalyst comprises from 98 to 100% by weight of copper oxide and zirconium oxide and from 2 to 0% by weight of metal oxides of transition groups Ib to VIIb and VIII of the Periodic Table of the Elements.

5. A process as claimed in claim 1, wherein the catalyst comprises from 99.5 to 100% by weight of copper oxide and zirconium oxide and from 0.5 to 0% by weight of metal oxides of transition groups Ib to VIIb and VIII of the Periodic Table of the Elements.

6. A process as claimed in any of claims 1 to 5 for preparing amines of the formula I 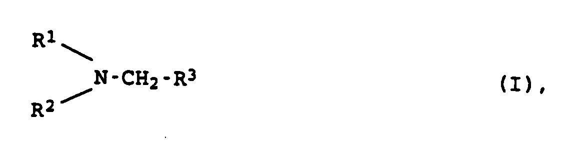 where
R¹ and R² are hydrogen, C₁-C₂₀-alkyl, C₃-C₁₂-cycloalkyl, aryl, C₇-C₂₀-aralkyl or C₇-C₂₀-alkylaryl,
R³ is C₁-C₂₀₀-alkyl, C₃-C₁₂-cycloalkyl, C₁-C₂₀-hydroxyalkyl, amino- and/or methylamino- and/or dimethylamino- and/or hydroxy-substituted C₁-C₂₀-alkyl, C₂-C₃₀-alkoxyalkyl, R⁴-(OCHR⁵CH₂)ₙ, aryl, C₇-C₂₀-aralkyl or C₇-C₂₀-alkylaryl,
R⁴ is hydrogen, C₁-C₄-alkyl,
R⁵ is hydrogen or methyl,
x is oxygen or N-R⁵,
n is an integer from 2 to 30,
l is an integer from 2 to 4,
m is an integer from 1 to 4,
from nitriles of the formula II
R³-CN (II)
and nitrogen compounds of the formula III

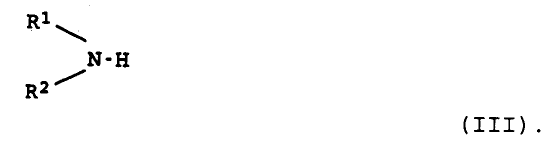

## Revendications

1. Procédé de préparation d'amines à partir de nitriles et de composés azotés, choisis dans le groupe formé par l'ammoniac, les amines primaires et secondaires, à des températures de 80 à 250°C et sous des pressions de 1 à 400 bar, avec de l'hydrogène en présence d'un catalyseur, caractérisé en ce que l'on utilise un catalyseur constitué de 85 à 100% en poids d'oxyde de cuivre et d'oxyde de zirconium et de 15 à 0% en poids d'oxydes métalliques d'éléments des groupes Ib à VIIb et VIII de la classification périodique des éléments.

2. Procédé selon la revendication 1, caractérisé en ce que le catalyseur est constitué de 90 à 100% en poids d'oxyde de cuivre et d'oxyde de zirconium et de 10 à 0% en poids d'oxydes métalliques d'éléments des groupes Ib à VIIb et VIII de la classification périodique des éléments.

3. Procédé selon la revendication 1, caractérisé en ce que le catalyseur est constitué de 95 à 100% en poids d'oxyde de cuivre et d'oxyde de zirconium et de 5 à 0% en poids d'oxydes métalliques d'éléments des groupes Ib à VIIb et VIII de la classification périodique des éléments.

4. Procédé selon la revendication 1, caractérisé en ce que le catalyseur est constitué de 98 à 100% en poids d'oxyde de cuivre et d'oxyde de zirconium et de 2 à 0% en poids d'oxydes métalliques d'éléments des groupes Ib à VIIb et VIII de la classification périodique des éléments.

5. Procédé selon la revendication 1, caractérisé en ce que le catalyseur est constitué de 99,5 à 100% en poids d'oxyde de cuivre et d'oxyde de zirconium et de 0,5 à 0% en poids d'oxydes métalliques d'éléments des groupes Ib à VIIb et VIII de la classification périodique des éléments.

6. Procédé selon l'une quelconque des revendications 1 à 5 pour la préparation d'amines de formule générale I 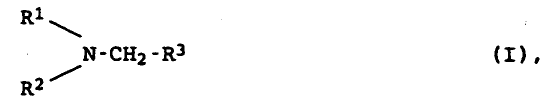 dans laquelle
R¹ et R² représentent un atome d'hydrogène, un groupement alkyle en C₁-C₂₀, cycloalkyle en C₃-C₁₂, aryle, aralkyle en C₇-C₂₀ et alkylaryle en C₇-C₂₀,
R³ représente un groupement alkyle en C₁-C₂₀₀, cycloalkyle en C₃-C₁₂, hydroxyalkyle en C₁-C₂₀, alkyle en C₁-C₂₀ substitué par des groupements amino et/ou méthylamino et/ou diméthylamino et/ou hydroxy, alcoxyalkyle en C₂-C₃₀, R⁴-(OCHR⁵CH₂)ₙ, aryle, aralkyle en C₇-C₂₀ et alkylaryle en C₇-C₂₀,
R⁴ représente un atome d'hydrogène, un groupement alkyle en C₁-C₄
R⁵ représente un atome d'hydrogène ou un groupement méthyle,
x représente un atome d'oxygène ou N-R⁵,
n représente un nombre entier de 2 à 30,
l représente un nombre entier de 2 à 4
m représente un nombre entier de 1 à 4
à partir de nitriles de formule générale II
R³-CN
et de composés azotés de formule générale III
